# EUROPÄISCHE PATENTSCHRIFT

(11) **EP 1 715 834 B1**
(45) Veröffentlichungstag und Bekanntmachung des Hinweises auf die Patenterteilung: **10.06.2009**
(21) Anmeldenummer: 05707273.8
(22) Anmeldetag: 09.02.2005
(51) Int. Cl.: A61K 8/06, A61K 8/60, A61K 8/02, A61Q 19/10

(54) **MIKROEMULSIONEN**
MICROEMULSIONS
MICRO-EMULSIONS

(30) Priorität: 18.02.2004 DE 102004008107
(43) Veröffentlichungstag der Anmeldung: 02.11.2006
(73) Patentinhaber: Cognis IP Management GmbH, 40589 Düsseldorf (DE)
(72) Erfinder: MÜLLER, Michael, 40789 Monheim (DE); BEHLER, Ansgar, 46240 Bottrop (DE); SEIDLER, Stefanie, 40597 Düsseldorf (DE); GOGET, Caroline, 40213 Düsseldorf (DE); ISSBERNER, Ulrich, 19002 Ambler (US)
(86) Internationale Anmeldenummer: PCT/EP2005/001279
(87) Internationale Veröffentlichungsnummer: WO 2005/089710

(56) Entgegenhaltungen:
- EP-A- 1 264 633
- WO-A-00/72952
- US-A- 4 252 826
- US-B1- 6 689 371
- FORGIARINI, ET AL.: "Studies of the relation between phase behavior and emulsification methods with nanoemulsion formation" PROGR. COLLOID POLYMER SCIENCE, Bd. 115, 2000, Seiten 36-39,

## Beschreibung

Die Erfindung befindet sich auf dem Gebiet der tensidischen Haar- und Hautbehandlungsmittel und Verfahren zur Herstellung dieser Mittel.

### Stand der Technik

An kosmetische Reinigungszubereitungen werden vom Verbraucher immer höhere Ansprüche gestellt. So sollen derartige Zubereitungen nicht nur über hervorragendes Schaumverhalten und gute Reinigungsleistung verfügen, sondern auch Haut und Haar pflegen und konditionieren. Bei den pflegenden Wirkstoffen oder Hautkonditioniemlitteln handelt es sich meistens um ölige Substanzen. Ölhaltige wässrige Tensidformulierungen zeigen üblicherweise ein schlechtes Schaumverhalten. Hierunter ist sowohl ein schlechtes Anschäumen als auch eine niedrige maximale Schaummenge zu verstehen. Daher werden sehr häufig ethoxylierte Verbindungen, wie z.B. Natriumlaurethsulfat, als Tensidkomponente verwendet. Da diese Verbindungen jedoch immer mehr in Verruf geraten, besteht eine große Nachfrage nach Formulierungen, die frei von ethoxylierten Verbindungen sind. Außerdem erfreuen sich transparente tensidische Zubereitungen größter Beliebtheit, wobei es sich als schwierig erwiesen hat, diese Transparenz auch bei Zusatz von Ölkörpern aufrecht zu erhalten.

So beschreibt die WO 98/40044 wäßrige Zubereitungen wasserlöslicher Tenside, die Lipid-Tensid-Mischmizellen mit einer mittleren Teilchengröße von unter 500 nm aufweisen und dabei weiß-bläulich erscheinen. Gegenstand der WO 98/15255 sind Mikroemulsionsgele vom Typ Ölin-Wasser, bei denen die Öltröpfchen in der Wasserphase durch assoziative Verdicker stabilisiert werden.

WO 00/72952 offenbart Emulsionen als Reinigungszubereitung , dei Verbindungen gemäß vorliegender Formel (I) enthalten.

Die Aufgabe der vorliegenden Erfindung hat somit darin bestanden, wäßrige transparente tensidische Formulierungen mit einem Gehalt an Ölkörpem zur Verfügung zu stellen, die ein hohes Schaumvolumen, gutes Anschäumverhalten und hohe Reinigungswirkung aufweisen. Des weiteren sollen die Formulierungen frei von ethoxylierten Verbindungen sein.

### Beschreibung der Erfindung

Gegenstand der vorliegenden Erfindung sind Mikroemulsionen mit einem mittleren Teilchendurchmesser von 5 bis 20 nm enthaltend (a) 5 bis 50 Gew.% wenigstens eines Alkyl- und/oder Alkenyloligoglykosidcarbonsäuresalzes gemäß Formel **(I),**

**R¹O[G]ₚO[(CH₂)ₘCOO⁻X⁺]ₙ** (I)

in der R¹ für einen Alkyl- und/oder Alkenylrest mit 4 bis 22 Kohlenstoffatomen, G für einen Zuckerrest mit 5 oder 6 Kohlenstoffatomen, p für Zahlen von 1 bis 10, m für Zahlen von 1 bis 5, n für Zahlen von 1 bis 5 und X für Alkali, Erdalkali, Ammonium, Alkanolammonium, Alkylammonium oder Glucammonium steht,(b) 5 bis 50 Gew.% einer Ölkomponente und (c) 0 bis 15 Gew. % mono- und/oder polyfunktioneller Alkohole mit 1 bis 4 Kohlenstoffatomen, wobei die Summe der Komponenten (a) +(b) 10 bis 55 Gew.% der Gesamtzusammensetzung ausmacht.

Als Tensidkomponente (a) wenigstens ein Alkyl- und/oder Alkenyloligoglykosidcarbonsäuresalz gemäß Formel (I)

R¹O[G]ₚO[(CH₂)ₘCOO⁻X⁺]ₙ (I)

in der R¹ für einen Alkyl- und/oder Alkenylrest mit 4 bis 22 Kohlenstoffatomen, G für einen Zuckerrest mit 5 oder 6 Kohlenstoffatomen, p für Zahlen von 1 bis 10, m für Zahlen von 1 bis 5, n für Zahlen von 1 bis 5 und X für Alkali, Erdalkali, Ammonium, Alkanolammonium, Alkylammonium oder Glucammonium steht, eingesetzt. Vorzugsweise steht R¹ für einen Alkyl-und/oder Alkenylrest mit 12 bis 18 und insbesondere für 12 bis 14 und/oder 16 bis 18 Kohlenstoffatome, n für Zahlen von 1 bis 3.

Sie können nach den einschlägigen Verfahren der präparativen organischen Chemie erhalten werden, beispielsweise durch Umsetzung von Alkyl- und/oder Alkenyloligoglykosiden mit Halogencarbonsäuren im alkalischen Medium in Gegenwart von Lösungsmitteln. Die Alkyl-und/oder Alkenyloligoglykosidcarbonsäure-Salze können sich von Aldosen bzw. Ketosen mit 5 oder 6 Kohlenstoffatomen, vorzugsweise der Glucose ableiten. Die bevorzugten Alkyl- und/oder Alkenyloligoglykosidcarbonsäure-Salze sind somit Salze von Alkyl- und/oder Alkenyloligoglucosidcarbonsäuren. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während q in einer gegebenen Verbindung stets ganzzahlig sein muss und hier vor allem die Werte p =1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Salze von Alkyl- und/oder Alkenyloligoglykosidcarbonsäuren mit einem mittleren Oligomerisierungsgrad p von 1,1 bis 3,0 eingesetzt. Aus anwendungstechnischer Sicht sind solche Alkyl- und/oder Alkenyloligoglykosidcarbonsäure-Salze bevorzugt, deren Oligomerisierungsgrad kleiner als 1,7 ist und insbesondere zwischen 1,2 und 1,4 liegt.

Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelenschen Oxosynthese erhalten werden. Der Alkyl- bzw. Alkenylrest R¹ leitet sich insbesondere von primären Alkoholen mit 12 bis 22, vorzugsweise 12 bis 18 und insbesondere 12 bis 14 und 16 bis 18 Kohlenstoffatomen ab. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucosid-ethercarbonsäue-Salze auf Basis von gehärtetem C_{12/14}-Kokosalkohol und C_{16/18}-Fettalkohol mit einem DP von 1 bis 3.

Die Alkyl- und/oder Alkenyloligoglykosidcarbonsäure-Salze leiten sich weiterhin vorzugsweise von Carbonsäuren, deren Salzen oder Estern ab, in der m für Zahlen von 1 bis 5, vorzugsweise 2 bis 4 und insbesondere 1 und 2, n für Zahlen von 1 bis 5 und vorzugsweise 1 bis 3 und X beispielsweise für Kalium, Ammonium, Triethanolammonium und vorzugsweise Natrium steht. Als Carbonsäuren, deren Salze oder Ester kommen als dem Fachmann bekannte Verbindungen und vorzugsweise Essigsäure, deren Salze, insbesondere Natrium oder Kaliumsalze, oder deren Ester, vorzugsweise mit 1 bis 4 Kohlenstoffatomen, in Frage. In einer bevorzugten Ausführungsform der Erfindung können die Alkyl- und/oder Alkenyloligoglykosidcarbonsäure-Salze durch Umsetzung einer wässrigen Lösung aus Alkyl- und/oder Alkenyloligoglycosiden (beispielsweise 20 bis 70 Gew.-%ige Lösungen - bezogen auf den Aktivsubstanzgehalt) in Stickstoffatmosphäre und in Gegenwart von Alkali, beispielsweise Alkalihydroxide oder Alkalicarbonate, bei 50 bis 100 °C mit ω-Halogencarbonsäure, deren Salz oder Ester, vorzugsweise Kalium- oder Natriummonochloracetat (MCA) erhalten werden. Vorzugsweise setzt man das Alkyl- und/oder Alkenyloligoglycosid mit der ω-Halogencarbonsäure, deren Salz oder Ester, vorzugsweise Kalium- oder Natriummonochloracetat (MCA), im Molverhältnis 1 : 0,5 bis 1 : 5 und vorzugsweise 1 : 1 bis 1 : 3 um. Weiterhin wählt man vorzugsweise ein Molverhältnis Alkali : ω -Halogencarbonsäure, deren Salz oder Ester von 1 : 0,5 bis 1 : 1,5 und vorzugsweise 1 : 1,1. Die Umsetzung von C_{12/14}-Alkyl- und/oder Alkenyloligoglycosiden erfolgt vorzugsweise ohne Zugabe organischer Lösungsmittel. C_{16/18}-Alkyl- und/oder Alkenyloligoglycosidcarbonsäure-Salze werden vorzugsweise in Gegenwart von C_{16/18}-Fettalkoholen sowie insbesondere 1,2 Propylenglycol hergestellt.
Derartige Alkyl- und/oder Alkenyloligoglykosidcarbonsäure-Salze zeichnen sich durch hohe Schaumstärke und ihre Milde gegenüber Haut und Haaren aus.

In einer weiteren besonders bevorzugten Ausführungsform wird als Tensidkomponente (a) eine Mischung aus wenigstens einem Alkyl- und/oder Alkenyloligoglykosidcarbonsäuresalz gemäß Formel (I)

**R¹O[G]ₚO[(CH₂)ₘCOO⁻X⁺]ₙ** (I)

in der R¹ für einen Alkyl- und/oder Alkenylrest mit 4 bis 22, vorzugsweise 12 bis 18 und besonders bevorzugt 12 bis 14 und/oder 16 bis 18 Kohlenstoffatomen, G für einen Zuckerrest mit 5 oder 6 Kohlenstoffatomen, p für Zahlen von 1 bis 10, m für Zahlen von 1 bis 5, n für Zahlen von 1 bis 5 und X für Alkali, Erdalkali, Ammonium, Alkanolammonium, Alkylammonium oder Glucammonium steht, und ein Tensid eingesetzt, welches ausgewählt ist aus der Gruppe, die gebildet wird von anionischen, kationischen, nichtionischen, zwitterionischen und amphoteren Tensiden.

### Anionische Tenside

Typische Beispiele für anionische Tenside sind Seifen, Alkylbenzolsulfonate, Alkansulfonate, Olefinsulfonate, α-Methylestersulfonate, Sulfofettsäuren, Alkylsulfate, Alkylethersulfate, Mono-und Dialkylsulfosuccinate, Mono- und Dialkylsulfosuccinamate, Sulfotriglyceride, Monoglyceridsulfate, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate und Proteinfettsäurekondensate (insbesondere pflanzliche Produkte auf Weizenbasis). Im Sinne der vorliegenden Erfindung sind Acylglutamate und ihre Salze und Alkyl- und/oder Alkenylsulfate besonders bevorzugt.

### Kationische Tenside

Typische Beispiele für kationische Tenside sind quartäre Ammoniumverbindungen und Esterquats, insbesondere quaternierte Fettsäuretrialkanolaminestersalze.

### Nichtionische Tenside

Typische Beispiele für nichtionische Tenside sind Alk(en)yloligoglykoside, Fettsäure-N-alkylglucamide, Polyolfettsäureester, Zuckerester, Sorbitanester, Polysorbate, Alkoholethoxylate und Aminoxide. Vorzugsweise werden Alkyl- und/oder Alkenyloligoglucoside, die insbesondere der Formel **(II)** folgen,

**R²O-[G]ₚ** (II)

in der R2 für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl-und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel **(II)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p =1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R² kann sich von primären Alkoholen mit 4 bis 11, vorzugsweise 8 bis 10 Kohlenstoffatomen ableiten. Typische Beispiele sind Butanol, Capronalkohol, Caprylalkohol, Caprin-alkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP =1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP =1 bis 3). Der Alkyl- bzw. Alkenylrest R² kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

Alkoholethoxylate werden herstellungsbedingt als Fettalkohol- oder Oxoalkoholethoxylate bezeichnet und folgen vorzugsweise der Formel **(III),**

**R¹O(CH₂CH₂O)ₙH** (III)

in der R¹ für einen linearen oder verzweigten Alkyl- und/oder Alkenylrest mit 6 bis 22 Kohlenstoff-atomen und n für Zahlen von 1 bis 50 steht. Typische Beispiele sind die Addukte von durchschnittlich 1 bis 50, vorzugsweise 5 bis 40 und insbesondere 10 bis 25 Mol an Capronalkohol, Caprylalkohol, 2-Ethylhexylalkohol, Caprinalkohol, Laurylalkohol, Isotridecylalkohol, Myristylalkohol, Cetylalkohol, Palm-oleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dime-risierung von ungesättigten Fettalkoholen anfallen. Bevorzugt sind Addukte von 10 bis 40 Mol Ethy-lenoxid an technische Fettalkohole mit 12 bis 18 Kohlenstoffatomen, wie beispielsweise Kokos-, Palm-, Palmkern-oder Talgfettalkohol.

### Zwitterionische und amphotere Tenside

Beispiele für geeignete amphotere bzw. zwitterionische Tenside sind Alkylbetaine, Alkylamidobetaine, Aminopropionate, Aminoglycinate, Imidazoliniumbetaine und Sulfobetaine. Beispiele für geeignete Alkylbetaine stellen die Carboxyalkylierungsprodukte von sekundären und insbesondere tertiären Aminen dar, die der Formel **(IV)** folgen, 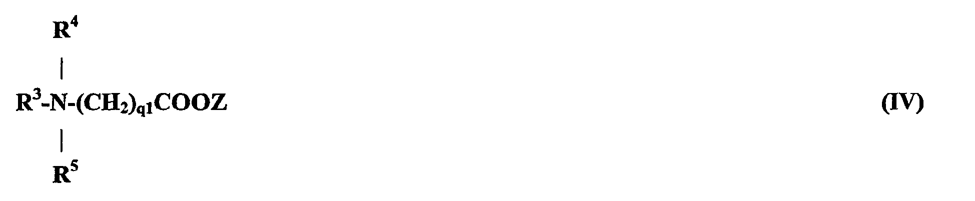 in der R3 für Alkyl- und/oder Alkenylreste mit 6 bis 22 Kohlenstoffatomen, R⁴ für Wasserstoff oder Alkylreste mit 1 bis 4 Kohlenstoffatomen, R⁵ für Alkylreste mit 1 bis 4 Kohlenstoffatomen, q1 für Zahlen von 1 bis 6 und Z für ein Alkali- und/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische.

Weiterhin kommen auch Carboxyalkylierungsprodukte von Amidoaminen in Betracht, die der Formel (V) folgen, 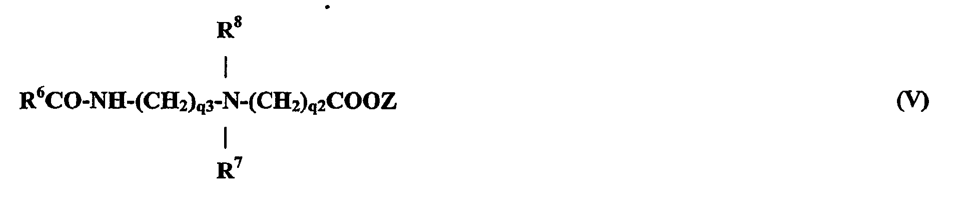 in der R⁶CO für einen aliphatischen Acylrest mit 6 bis 22 Kohlenstoffatomen und 0 oder 1 bis 3 Doppelbindungen, R⁷ für Wasserstoff oder Alkylreste mit 1 bis 4 Kohlenstoffatomen, R⁸ für Alkylreste mit 1 bis 4 Kohlenstoffatomen, q2 für Zahlen von 1 bis 6, q für Zahlen von 1 bis 3 und Z wieder für ein Alkali- und/oder Erdalkalimetall oder Ammonium steht. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat konden-siert werden. Bevorzugt ist der Einsatz eines Kondensationsproduktes von C_{8/18}-Kokosfettsäure-N,N-dime-thylaminopropylamid mit Natriumchloracetat.

Weiterhin kommen auch Imidazoliniumbetaine in Betracht. Auch bei diesen Substanzen handelt es sich um bekannte Stoffe, die beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum C_{12/14}-Kokosfettsäure, die anschließend mit Natriumchloracetat betainisiert werden.

Vorzugsweise wird die Tensidkomponente (a) in einer Menge von 10 bis 35 Gew.% bezogen auf die Gesamtformulierung eingesetzt.

Als Ölkomponente (b) dienen sowohl unpolare als auch polare Öle oder Mischungen hiervon. Hierzu zählen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane oder Silikonöle oder besonders bevorzugt Hydrogenated Polydecene.

Als Ölkomponente (b) können jedoch auch feste Fette und/oder Wachse. Diese können auch in Mischung mit den im vorherigen Abschnitt genannten Ölen vorliegen. Typische Beispiele für Fette sind Glyceride, d.h. feste oder flüssige pflanzliche oder tierische Produkte, die im Wesentlichen aus gemischten Glycerinestern höherer Fettsäuren bestehen. Hier sind insbesondere feste Mono- und Diglyceride zu nennen wie z.B. Glycerinmonooleat oder Glycerinmonostearat. Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage. Tocopherole und ätherische Öle eignen sich ebenfalls als Ölkomponente (b).

Vorzugsweise wird die Ölkomponente (b) in einer Menge von 10 bis 20 Gew.% bezogen auf die Gesamtzusammensetzung eingesetzt. Die Größe der dispergierten Teilchen liegt hierbei im Bereich von 5 bis 20 nm. Um die erfindungsgemäßen Zubereitungen zu erhalten, sollte die Summe aus Tensidkomponente und Ölkomponente in den Formulierungen bei 0,5 bis 55 Gew.%, vorzugsweise bei 2 bis 40 Gew. % liegen.

Fakultativ kann die Mikroemulsion mono- oder polyfunktionelle Alkohole mit 1 bis 4 Kohlenstoffatomen in einer Menge von 0 bis 15 Gew. % bezogen auf die Gesamtformulierung enthalten. Bevorzugt werden Alkohole der Gruppe, die gebildet wird von Ethanol, Glycerin, Ethylenglykol und/oder Propylenglykol. Durch die Zugabe dieser Alkohole wird die Aufnahmefähigkeit der Mikroemulsion für Öle erhöht. Außerdem kann der Brechungsindex der Wasserphase dem der dispergierten Ölphase angeglichen werden, so daß eine mögliche Trübung reduziert wird. Außerdem erhöht sich die Lagerstabilität der Emulsion bei niedrigen Temperaturen, z.B. bei -5°C.

Die Emulsionen lassen sich durch Polymere verdicken. Besonders bevorzugt sind in diesem Zusammenhang die als assoziative Verdicker bekannten Verbindungen wie PEG-120 Methylglucosedioleat oder PEG-150-Distearat.

Die Mikroemulsionen werden durch Verrühren aller Komponenten bei Raumtemperatur oder, vorzugsweise bei Einsatz von festen Komponenten, durch vorheriges Aufschmelzen und Homogenisieren der bei Raumtemperatur festen Komponenten und anschließendem Verrühren mit der tensidhaltigen Wasserphase in der Hitze hergestellt. Der Homogenisierungsprozess wird generell durch Erhitzen beschleunigt. Als geeignete Temperaturen haben sich Temperaturen im Bereich von 50 - 70°C herausgestellt. Für das Homogenisieren reicht ein einfaches Rührwerk aus. Da die Ölkörper (b) bei Raumtemperatur als feste Substanzen vorliegen können, kann es sich in diesen Fällen bei den resultierenden Emulsionen um Feststoff-Dispersionen handeln.

Die erfindungsgemäßen Mikroemulsionen können kalt mit anderen Formulierungen verrührt werden und bewerkstelligen auf diese Weise die Einarbeitung von Ölkörpem in kosmetische Formulierungen, die auf herkömmlichem Wege nicht erreicht werden können. Transparente ölhaltige kosmetische Reinigungsformulierungen sind auf diese Weise einfach erhältlich. Die erhaltenen Zubereitungen sind stabil und verfügen über ein hervorragendes Schaumverhalten. Die erfindungsgemäßen Mikroemulsionen weisen vorzugsweise einen Trübungswert von 1 bis 15 NTU auf und sind somit transparent.

Ein weiterer Gegenstand der vorliegenden Anmeldung sind Feuchttücher, die dadurch gekennzeichnet sind, daß sie mit einer erfindungsgemäßen Mikroemulsion imprägniert sind. Bei diesen Feuchttüchem handelt es sich um Flächegebilde auf Basis von Papier, Vliesen (nonwoven) oder Geweben (woven), die mit einer erfindungsgemäßen Mikroemulsion beschichtet sind und zur Körperpflege und -reinigung dienen. Damit die Mikroemulsionen im großtechnischen Verfahren einfach appliziert werden können, ist es vorteilhaft derartige Mikroemulsionen für die Beschichtung zu verwenden, die einen Wassergehalt von mindestens 60 Gew. %, vorzugsweise 70 Gew. % und insbesondere bevorzugt mehr als 75 Gew. % bezogen auf die Gesamtzusammensetzung der Emulsion aufweisen. Die derartig ausgerüsteten Flächengebilde können in einem Trocknungsschritt nachbehandelt werden, um den Wassergehalt nach der Applikation zu reduzieren oder um nahezu wasserfreie Produkte zu erhalten (Dry Wipes).

### Beispiele

### Beispiele 1 und 2

| | **1** | **2** |
|---|---|---|
| Lauryl Glucose Carboxylate (and) Lauryl Glucoside (Plantapon^{®} LGC SORB) | 17,08 | 20,90 |
| Cocamidopropyl Betaine | 10,25 | - |
| Disodium Cocoyl Glutamate | - | 1,10 |
| Glyceryl Oleate | 3,66 | 5,68 |
| Dicaprylyl Ether | 6,76 | 9,23 |
| Octyldodecanol | 2,25 | - |
| Myristyl Myristate | - | 3,09 |
| Hydrogenated Polydecene | | |
| Glycerin | - | 5,00 |
| Wasser | 60 | 55,00 |
| Viskosität/mPas* | 24 | 35 |
| Partikelgöße** | 6,1 (a) | 7,4 (a) |
| Trübung / NTU*** | 2,01 | 3,12 |

| | | |
|---|---|---|
| * Kegel-Platte Viskosimeter (C-VOR Bohlin Instruments), T=25°C, Scherrate 10s⁻¹ ** Mittelwert Teilchendurchmesser in nm; (a): gemessen mit Horiba LB-500 Partikelgrößenmessgerät (Prinzip: Dynamische Lichtstreuung); (b) gemessen mit Coulter LS 230 Partikelgrößenmessgerät (Prinzip: Laserbeugung) *** Gemessen mit einem Nephelometer, welches als Lichtquelle für weißes Licht eine Wolframglühfadenlampe benutzt. Gerät: Labortrübungsmessgerät 2100 AN IS von HACH Company. Messeinheit NTU | | |

Bei den Zubereitungen 1 und 2 handelt es sich um eine transparente niedrigviskose Mikroemulsion mit bemerkenswerten Schaumeigenschaften.

### Schaumkinetik zu Beispiel 1:

Messmethode Rotorschaum (Messgerät: Sita Foam Tester R-2000), Umdrehungszahl Rotor 1300 rpm, pH =6, Messtemperatur 40°C, Konzentration: Mikroemulsion wurde auf 3g/l Aktivsubstanz verdünnt (Aktivsubstanz =Alle Komponenten außer Wasser). Wasserhärte: 15°dH.

| | | | | | | | | | |
|---|---|---|---|---|---|---|---|---|---|
| Rührzeit/Sekunden | 0 | 30 | 60 | 90 | 120 | 140 | 150 | 160 | 170 |
| Schaumvolumen / ml | 0 | 250 | 400 | 510 | 640 | 730 | 750 | 770 | 770 |

An der Schaumkinetik des Beispiels 1 wird deutlich, dass die erfindungsgemäßen Mikroemulsionen relativ schnell sehr stark aufschäumen.

### Beispiel 3

Die in den Beispielen 1 bis 2 erhaltenen Mikroemulsionen wurden bei Raumtemperatur mit Wasser und Konservierungsstoffen versetzt. Die entstandenen Benetzungslösungen eigneten sich insbesondere als sprühbare Lotion für Reinigungstücher, vorzugsweise für das Gesicht und Babyhaut. Es wurden 3 g der Benetzungslösung pro g des Tuches durch Tränken oder Besprühen aufgetragen.

### Zusammensetzung der Benetzungslösungen:

| | |
|---|---|
| Mikroemulsion gemäß Beispielen 1 bis 2 | 20 Gew. % |
| Wasser | 79 Gew. % |
| Euxyl^{®} K702 | 1 Gew. % |
| Citronensäure | q.s. (pH =5,5) |

## Patentansprüche

1. Mikroemulsion mit einem mittleren Teilchendurchmesser von 5 bis 20 nm enthaltend
(a) 5 bis 50 Gew. % wenigstens eines Alkyl- und/oder Alkenyloligoglykosidcarbonsäuresalzes gemäß Formel **(I),**
**R¹O[G]ₚO[(CH₂)ₘCOO⁻X⁺]ₙ** (I)
in der R¹ für einen Alkyl- und/oder Alkenylrest mit 4 bis 22, G für einen Zuckerrest mit 5 oder 6 Kohlenstoffatomen, p für Zahlen von 1 bis 10, m für Zahlen von 1 bis 5, n für Zahlen von 1 bis 5 und X für Alkali, Erdalkali, Ammonium, Alkanolammonium, Alkylammonium oder Glucammonium steht,
(b) 5 bis 50 Gew.% einer Ölkomponente und
(c) 0 bis 15 Gew.% mono- und/oder polyfunktioneller Alkohole mit 1 bis 4 Kohlenstoffatomen,
wobei die Summe der Komponenten (a) +(b) 10 bis 55 Gew.% der Gesamtzusammensetzung ausmacht.

2. Mikroemulsion gemäß Anspruch 1, **dadurch gekennzeichnet, dass** die Emulsion weiterhin ein Tensid enthält, welches ausgewählt ist aus der Gruppe, die gebildet wird von anionischen, kationischen, nichtionischen, amphoteren und zwitterionischen Tensiden sowie deren Mischungen.

3. Mikroemulsion gemäß wenigstens einem der Ansprüche 1 und/oder 2, **dadurch gekennzeichnet, dass** als Komponente (c) Ethanol, Glycerin, Ethylenglykol und/oder Propylenglykol eingesetzt wird.

4. Feuchttücher, **dadurch gekennzeichnet, dass** sie mit einer Mikroemulsion gemäß mindestens einem der Ansprüche 1 bis 3 imprägniert sind.

5. Feuchttücher, **dadurch gekennzeichnet, dass** sie mit einer Mikroemulsion gemäß mindestens einem der Ansprüche 1 bis 3 imprägniert ist, wobei die Mikroemulsion vorher auf einen Wassergehalt von mindestens 60 Gew. % verdünnt wurde.

## Claims

1. Microemulsion with a mean particle diameter of 5 to 20 nm containing
(a) 5 to 50% by weight of at least one alkyl and/or alkenyl oligoglycoside carboxylic acid salt corresponding to formula (I):
**R¹O[G]ₚO[(CH₂)ₘCOO⁻X⁺]ₙ** (I)
in which R¹ is an alkyl and/or alkenyl group containing 4 to 22 carbon atoms, G is a sugar unit containing 5 or 6 carbon atoms, p is a number of 1 to 10, m is a number of 1 to 5, n is a number of 1 to 5 and X stands for alkali metal, alkaline earth metal, ammonium, alkanolammonium, alkyl ammonium or glucammonium,
(b) 5 to 50% by weight of an oil component and
(c) 0 to 15% by weight of mono- and/or polyhydric alcohols containing 1 to 4 carbon atoms,
the sum of components (a) + (b) making up 10 to 55% by weight of the composition as a whole.

2. Microemulsion as claimed in claim 1, **characterized in that** the emulsion additionally contains a surfactant selected from the group consisting of anionic, cationic, nonionic, amphoteric and zwitterionic surfactants and mixtures thereof.

3. Microemulsion as claimed in at least one of claims 1 and/or 2, **characterized in that** ethanol, glycerol, ethylene glycol and/or propylene glycol is used as component (c).

4. Wet wipes, **characterized in that** they are impregnated with the microemulsion claimed in at least one of claims 1 to 3.

5. Wet wipes, **characterized in that** they are impregnated with the microemulsion claimed in at least one of claims 1 to 3, the microemulsion having been diluted beforehand to a water content of at least 60% by weight.

## Revendications

1. Microémulsion présentant un diamètre moyen des particules de 5 à 20 nm contenant
(a) 5 à 50% en poids d'au moins un sel d'un acide alkyloligoglycosidecarboxylique et/ou alcényloligoglycosidecarboxylique selon la formule (I),
R¹O[G]ₚO(CH₂)ₘCOO⁻X⁺]ₙ (I)
dans laquelle R¹ représente un radical alkyle et/ou alcényle comprenant 4 à 22 atomes de carbone, G représente un radical de sucre comprenant 5 ou 6 atomes de carbone, p représente des nombres de 1 à
10, m représente des nombres de 1 à 5, n
représente des nombres de 1 à 5 et X représente un métal alcalin, un métal alcalino-terreux, ammonium, un alcanolammonium, un alkylammonium ou glucammonium,
(b) 5 à 50% en poids d'un composant huileux et
(c) 0 à 15% en poids d'alcools monofonctionnels et/ou polyfonctionnels comprenant 1 à 4 atomes de carbone,
la somme des composants (a) + (b) représentant 10 à 55% en poids de la composition totale.

2. Microémulsion selon la revendication 1, **caractérisée en ce que** l'émulsion contient en outre un agent tensioactif, qui est choisi dans le groupe formé par les agents tensioactifs anioniques, cationiques, non ioniques, amphotères et zwittérioniques, ainsi que leurs mélanges.

3. Microémulsion selon au moins l'une quelconque des revendications 1 et/ou 2, **caractérisée en ce qu'**on utilise comme composant (c) l'éthanol, le glycérol, l'éthylèneglycol et/ou le propylèneglycol.

4. Lingettes humides, **caractérisées en ce qu'**elles sont imprégnées par une microémulsion selon au moins l'une quelconque des revendications 1 à 3.

5. Lingettes humides, **caractérisées en ce qu'**elles sont imprégnées par une microémulsion selon au moins l'une quelconque des revendications 1 à 3, la microémulsion étant diluée au préalable à une teneur en eau d'au moins 60% en poids.
